# EUROPEAN PATENT APPLICATION

(11) **EP 4 609 863 A1**
(43) Date of publication of application: **03.09.2025**
(21) Application number: 23893252.9
(22) Date of filing: 01.08.2023
(51) Int. Cl.: A61K 31/4709, A61K 9/16, A61K 9/20, A61K 9/48, A61K 9/08, A61K 9/10, A61P 13/12

(54) **USE OF LY2922470 IN PREPARING A MEDICAMENT FOR PREVENTING OR TREATING RENAL DISEASES**

(30) Priority: 22.11.2022 CN 202211466949
(71) Applicant: Jeamoon Beijing Co., Ltd., Beijing 100098 (CN)
(72) Inventor: CUI, Qinghua, Beijing 100098 (CN); ZHOU, Wanlu, Beijing 100098 (CN)
(74) Representative: Chung, Hoi Kan
(86) International application number: PCT/CN2023/110567
(87) International publication number: WO 2024/109153

(57) **Abstract**

Provided is use of LY2922470 in preparing a medicament for preventing or treating renal diseases. The therapeutic effects of LY2922470 and TAK875 on renal failure were evaluated in an adenine-induced mouse renal failure model. The results show that LY2922470 has the effect of treating renal failure, and the therapeutic effect has no direct relationship with the GPR40 target. A renal fibrosis in-vitro model also demonstrates that LY2922470 has an inhibitory effect on renal fibrosis.

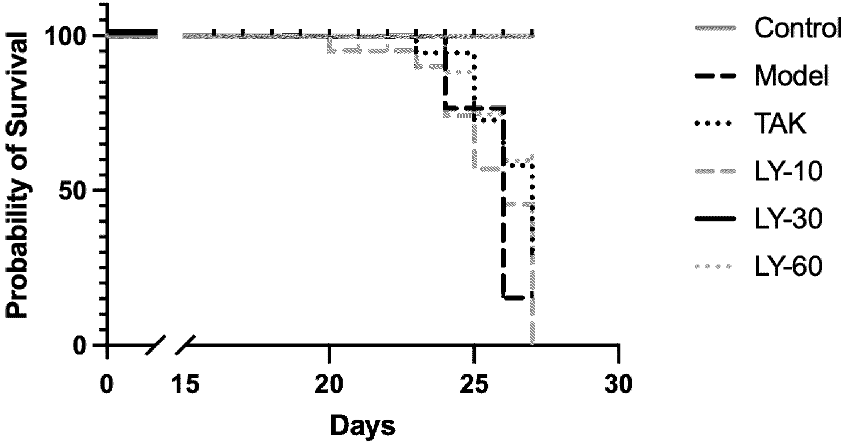

## Description

### Technical Field

The present invention belongs to the field of biomedicine and specifically relates to a use of LY2922470 in preparing a medicament for preventing or treating renal diseases.

### Background Technology

LY2922470 was developed by Eli Lily and Company as a small molecule activator of GPR40 for the treatment of type 2 diabetes mellitus, and comprises following structural formula:

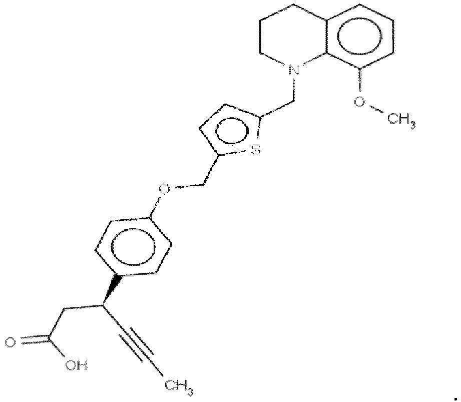

GPR40 belongs to the GPCR family. The activation of GPR40 has been proven to induce pancreatic β cells to secrete glucose-stimulated insulin (GSIS) and intestinal endocrine cells to secrete enterotropic insulin. Therefore, GPR40 has become a feasible and promising therapeutic target for type 2 diabetes mellitus without the risk of hypoglycemia. Thus, it has attracted considerable attention as a therapeutic medicament target for type 2 diabetes mellitus (T2DM). Currently, the anti-diabetic effects of many GPR40 ligands have been developed and studied. Among them, TAK-875, as a GPR40 activator, has been successfully tested in Phase II clinical trials.

The general formula of the compound structure of LY2922470 was first proposed in patent WO2015105786A1, and it was suggested that this compound could be used to treat type 2 diabetes mellitus.

Renal failure, also known as RF for short, is a syndrome composed of decreased glomerular filtration rate (GFR) caused by chronic renal diseases due to various reasons, as well as related metabolic disorders and clinical symptoms, which is characterized by the continuous and progressive decline of glomerular filtration rate.

The incidence of chronic renal failure in China is over five per ten thousand of the total population. Besides the expensive hemodialysis and renal transplantation, there is currently a lack of effective treatment medicaments. Therefore, seeking new medicaments for treating renal diseases is of great significance.

Renal fibrosis is regarded as a common pathway of almost all types of chronic renal diseases (CKD), and it has an impact on glomeruli, renal tubules, renal vessels, etc. Fibrosis is also a hallmark of CKD, characterized by epithelial-mesenchymal transition (EMT), excessive extracellular matrix (ECM) deposition, and accumulation of mesenchymal cells and inflammatory cells. Through EMT, tubular epithelial cells become myofibroblasts, which have enhanced cell proliferation, movement, contraction, and the ability of excessive ECM deposition, thereby leading to fibrosis. Injury such as ischemia-reperfusion (IR), nephrotoxins, chemotherapy medicaments, diabetes, hypertension and ureteral obstruction can all lead to the occurrence of renal fibrosis.

Transforming growth factor -β1 (TGF-β1) can exert its function by regulating the TGF-β/Smad pathway, making it the overall regulator of fibrosis. Therefore, TGF-β1 can be used to induce in vitro fibrosis models.

### Summary of the Invention

The technical problem to be solved by the present invention is to provide a new type of indication for the medicament LY2922470. LY2922470, as a medicament for renal diseases, can effectively improve renal function and provide a new alternative medicament for the prevention and treatment of renal injury, chronic renal diseases and renal failure.

The present invention provides following technical solutions: in a use of LY2922470 in preparing a medicament for preventing or treating renal diseases, the LY2922470 comprising following structural formula:

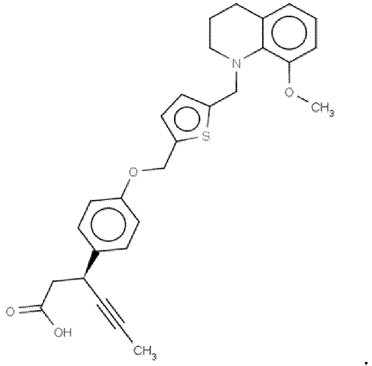

Further, the renal diseases comprise renal injury, chronic rental diseases, or renal failure

Further, the renal diseases comprise renal diseases caused by renal fibrosis.

Further, the renal diseases comprise nephropathy caused by ischemia-reperfusion (IR), nephrotoxins, chemotherapeutic medicaments, hypertension or ureteral obstruction or diabetic nephropathy.

Further, the medicament comprises the LY2922470 exists in a form of a compound or a pharmaceutically acceptable salt thereof.

Further, the medicament is in an oral or injectable dosage form, which comprises powders, tablets, granules, capsules, oral liquids, emulsions or suspensions.

Further, the medicament further comprises auxiliary materials or medicines for protecting renals and blood vessels.

Further, the medicament further comprises a pharmaceutically acceptable carrier, and the pharmaceutically acceptable carrier comprises a diluent, a buffer, a suspension, an emulsion, a granule, an encapsulation, an excipient, a filler, an adhesive, a spray, a transdermal absorbent, a wetting agent, a disintegrant, an absorption accelerator, a surfactant, a colorant, a flavoring agent or an adsorption carrier.

In the present invention, the medicament contains an effective dose of LY2922470. The effective dose is in the form of a unit dose administered (such as the content in one tablet, one injection, one pill or one dose of the medicament) or the unit dose of patients being treated (such as the dose per unit body weight). In the present invention, the scope of the medicament treatment targets is mammalian animals, including humans, canines, rodents, etc. The effective dose conversion for different animals can be based on the equivalent dose conversion relationship between experimental animals and humans in this field (usually referring to the guidelines of medicament regulatory agencies such as the FDA and SFDA, or also to "Huang Jihan et al. Equivalent dose conversion between animals and between animals and humans in pharmacological experiments. ; The dose per unit body weight of humans can be derived from the dose of experimental animals (9 (9) : 1069-1072 "). For instance, for the commonly used experimental animal, mice, according to the above-mentioned literature, the conversion relationship between them and adults is approximately 12:1.

In the present invention, in 8-week-old C57BL/6 N mice, the effective dose (by content) for the obvious treatment of adenine-induced renal failure was 20-100 mg/kg, preferably 30-60 mg/kg.

Preferably, based on the conversion relationship between the effective doses of mice and adults, if the adult body weight standard is set at 60 kg, then the effective dose for adults is 100 to 500 mg per day, preferably 150 to 300 mg.

Compared with prior art, the present invention has following beneficial effects:
The present invention through the adoption of adenine LY2922470 induced renal failure animal model evaluation, TAK875 treatment effect of renal injury and renal failure, the experimental results show that the LY2922470 have the function of the treatment of renal failure, and the therapeutic effect and GPR40 targets has no direct relation. The in vitro model of renal fibrosis confirmed that LY2922470 has an inhibitory effect on renal fibrosis. The present invention confirms the significant potential of LY2922470 as a medicament for renal diseases, discloses for the first time that LY2922470 effectively improves renal function, provides an effective new potential alternative medicament for the treatment of renal injury, chronic renal diseases and renal failure, expands the indications of LY2922470, and has greatly enhanced the application potential and market prospects of LY2922470.

### Brief Description of the Drawings

Figure 1 shows glomerular filtration rates of mice that were modeled after being fed with a model diet containing 0.25% adenine for 3 consecutive weeks.
Figure 2 shows changes of serum creatinine (CREA) in an adenine-induced mouse renal failure model (*P<0.05, **P<0.01, ***P<0.0001).
Figure 3 shows survival curves of LY2922470 in treatment of adenine-induced renal failure in mice.
Figure 4 shows glomerular filtration rates of mice with continuous adenine-induced renal failure treated with LY2922470.
Figure 5 shows glomerular filtration rates of mice with adenine-induced renal failure for two weeks treated with LY2922470.
Figure 6 shows a statistical graph of degree of renal fibrosis in mice treated with LY2922470 for adenine-induced renal failure (MASSON staining semi-quantitative analysis).
Figure 7 shows RT-PCR quantitative results of TGF-β 1-induced HK-2 cell fibrosis markers treated with LY2922470.
Figure 8 shows RT-PCR quantitative results of fibrosis markers of HK-2 cells induced by high glucose treated with LY2922470.

### Specific Embodiments

The experimental methods in following embodiments, unless otherwise specified, are all conventional methods. The experimental materials used in the following embodiments, unless otherwise specified, can all be purchased from commercial channels.

**Embodiment 1:** Adenine-induced renal injury and renal failure in mice was successfully constructed.

### I. Experimental Materials

i. Experimental animals: C57BL/6N, 8 weeks old, SPF grade, male, provided by Spef (Beijing) Biotechnology Co., LTD.

### II. Modeling Methods for Mouse Models of Renal Injury and Renal Failure (Adenine Induction)

Except for a blank control group, the remaining mice were fed with model feed containing 0.25% adenine for modeling. The blank control group was fed with normal feed, and the mice ate freely for three weeks. At the end of the third week, glomerular filtration rate detection and related renal function index detection were conducted.

### III. Experimental results and conclusions

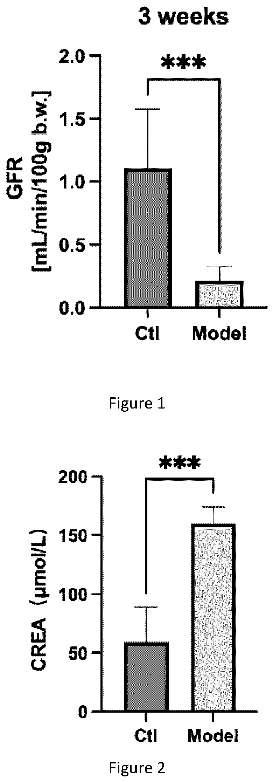

The glomerular filtration rates of mice in the blank control group and the adenine-induced renal failure model group are shown in figure 1. Compared with mice fed with normal feed, the glomerular filtration rates of mice fed with feed containing 0.25% adenine decreased significantly (P<0.001), and the glomerular filtration function was basically lost. The concentration change of serum creatinine (CREA) is mainly determined by the glomerular filtration capacity (glomerular filtration rate). The filtration capacity decreases and the creatinine concentration increases. Serum creatinine can accurately reflect the condition of renal parenchymal damage. The CREA of the blank control group and the model group was shown in figure 2. Compared with the blank control group, the CREA of mice in the model group was significantly upregulated (P<0.001). The physiological and pathological manifestations of mice with renal failure were consistent, and the renal failure model was successfully constructed.

**Embodiment 2:** LY2922470 effectively reduces the mortality rate of mice caused by renal failure.

### I. Experimental Materials

i. Experimental animals: C57BL/6N, 8 weeks old, SPF grade, male.
ii. Experimental drugs: LY2922470 (purchased from Shanghai Taoshu Biology Co., LTD.), and TAK875 (purchased from Shanghai Taoshu Biology Co., LTD.).
   i. Preparation Method for TAK875: Weigh 27 mg of TAK875 powder by using a precision balance, dissolve it in 9 ml of 0.8% CMC-Na solution, and sonicate for 5 minutes to obtain a white suspension at a concentration of 3 mg/ml. Store it at 4°C for later use.
   ii. Preparation Method for LY2922470: Add 80 mg of yellow crystalline LY2922470 to 200 µl of DMSO solution to create a yellow transparent solution. Then, gradually add this solution to 10 ml of 0.8% CMC-Na solution, and heat-sonicate for 5 hours to prepare a white suspension of LY2922470 at a concentration of 8 mg/ml. Store it at 4°C for later use.

### II. Experimental Grouping and Drug Administration

| Group | Number of Animal (n) | Dosage | Method of Administration |
|---|---|---|---|
| Blank Control Group (Ctl) | 8 | - | p.o, QD |
| Model Group(Model) | 12 | - | p.o, QD |
| TAK875 Group | 12 | 30 mg/kg | p.o, QD |
| Low-dose LY Group | 12 | 10 mg/kg | p.o, QD |
| Medium-dose LY Group | 12 | 30 mg/kg | p.o, QD |
| High-dose LY Group | 12 | 60 mg/kg | p.o, QD |

| | | | |
|---|---|---|---|
| p.o: oral administration. QD: once daily. | | | |

### III. Experimental Procedures

Select 8-week-old male C57BL/6N mice and adapt them for one week. Randomly divide them into six groups. Except for the blank control group, the remaining mice were fed with model feed containing 0.25% adenine to establish the model, while the blank control group was fed normal feed. Mice had free access to food. One week after modeling, each group was administered drugs by gavage once daily in the afternoon. The blank control group and the model group received the solvent under the same conditions. During the administration period, continue feeding the adenine diet to the model group, and record the survival status of the mice until the end of the experiment.

### IV. Experimental Results and Conclusions

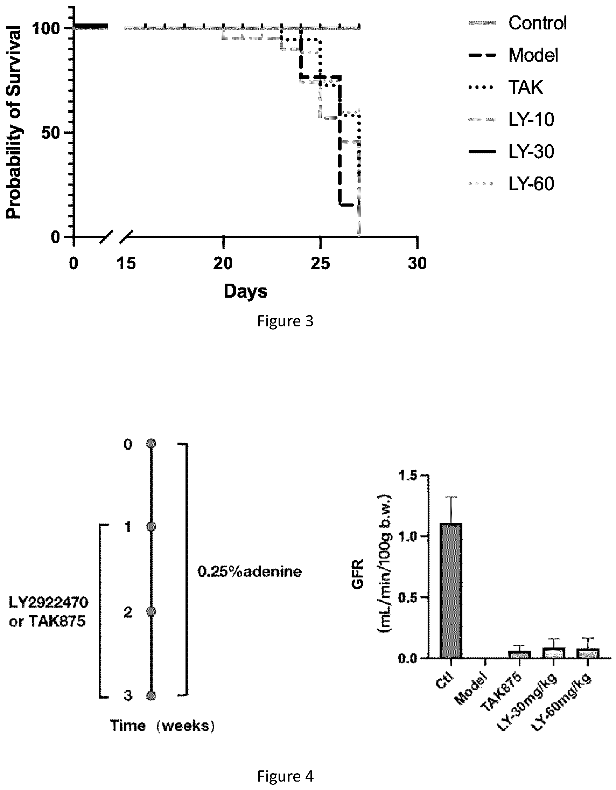

Table 1 shows the survival rate statistics of LY2922470 in the adenine-induced renal failure model in mice, and figure 3 presents the corresponding survival curves. No deaths occurred among the mice 19 days post-adenine feeding; however, all groups experienced weight loss, and the mice gradually became emaciated. Starting from day 20 of adenine feeding, mouse deaths began to occur, with all mice in the LY-10 mg/kg group dead by day 27. The survival rate of the blank control group was 100%, while the model group's survival rate was only 14.3%. The TAK875 treatment group had a survival rate of 46.20%, and the survival rates of the LY-60 mg/kg group and the TAK875 group were similar, at around 50%. LY2922470 exhibited the strongest protective effect on the renals at a dosage of 30 mg/kg, resulting in a final survival rate of 100%, which was double that of the TAK875 group.

From the data in following table, it can be seen that LY2922470 has therapeutic effects at dosages ranging from 30 mg/kg/d to 60 mg/kg/d, with the best therapeutic effect on renal failure observed at 30 mg/kg/d. However, the possibility of doses <30 mg/kg/d (such as 20-30 mg/kg) providing protective effects on the kidneys cannot be ruled out, nor can the therapeutic effects of doses >60 mg/kg/d (such as 60-100 mg/kg).

**Table 1**

| Survival Rate Statistics of LY2922470 in Adenine-Induced Mouse Renal Failure Model | | | |
|---|---|---|---|
| Group | Enrollment | End of Treatment | Survival Rate % |
| | Quantity (pieces) | | |
| Blank Control Group | 8 | 8 | 100% |
| Model Group | 14 | 2 | 14.30% |
| TAK875 Group | 13 | 6 | 46.20% |
| LY-10 mg/kg Group | 12 | 0 | 0% |
| LY-30 mg/kg Group | 12 | 12 | 100% |
| LY-60 mg/kg Group | 12 | 6 | 50% |

**Embodiment 3:** The therapeutic effect of LY2922470 on renal injury and renal failure

### I. Experimental Materials

i. Experimental Animals: C57BL/6N, 8 weeks old, SPF grade, male.
ii. Experimental Drugs: LY2922470, TAK875 (both purchased from Shanghai Taoshu Biological Co., Ltd.).

### II. Experimental Grouping

a) Blank Control Group: fed with adenine-free feed and the rest of the operations were the same as the model group.
b) Model Group (Model): fed with adenine-containing feed for modeling.
c) LY-10 mg/kg Group: Model + LY2922470 (10 mg/kg, p.o, QD).
d) LY-30 mg/kg Group: Model + LY2922470 (30 mg/kg, p.o, QD).
e) LY-60 mg/kg Group: Model + LY2922470 (60 mg/kg, p.o, QD),
f) TAK875 Group: Model + TAK875 (30 mg/kg, p.o, QD).

p.o: oral administration.
QD: once a day.

### III. Experimental Methods

### i. Experimental Procedures

Except for the blank control group, the remaining mice were fed with model feed containing 0.25% adenine for modeling. The blank control group was fed with normal feed, and the mice ate freely. One week after modeling, the drug was administered by gavage according to different groups once a day in the afternoon for two consecutive weeks. The blank control group and the model group were given the solvent under the same conditions. During the administration period, the model was continuously fed with adenine feed until the end of the experiment. During the experiment, the condition of the mice was observed. Their weights were measured and recorded every week. Blood samples were collected from the fundus at 1 week and 3 weeks (2 weeks after administration) after feed modeling respectively. Serum was separated and serum CRE and BUN were measured. At 2 weeks after administration, the TGFR renal function monitoring and intelligent analysis system was used to test the renal function of mice in each group. After the test, samples were taken from the mice for detection to confirm the efficacy of the drug.

### ii. Data Collection and Statistical Analysis

GraphPad software and Prism 8.0 statistical software were used for statistical analysis. The data of each group of measurement data were expressed as mean ± SD. ANOVA one-way analysis of variance was used for multiple group comparisons. The t-test was used for comparisons between two groups. P < 0.05 was considered to be statistically significant. The chi-square test was used for comparisons between groups of count data. P < 0.05 was considered to be statistically significant.

### IV. Experimental results and conclusions

### i. Glomerular Filtration Rate

Figure 4 shows that mice were continuously fed with 0.25% adenine, and LY2922470 or TAK875 was given after 1 week. During the administration period, the mice were fed with adenine feed to establish the model. After 3 weeks of adenine feeding (two weeks of administration), the TGFR renal function monitoring and intelligent analysis system was used to detect the glomerular filtration rate of mice.

As shown in figure 4, compared with the blank control group, the glomerular filtration rates of mice in the model group were significantly reduced, and the glomerular filtration function almost completely disappeared. Compared with the model group, the glomerular filtration rates of mice were improved after oral administration of the GPR40 agonist TAK875. After oral administration of different doses of LY2922470, the renal function of mice was improved, and the improvement of mice using 30 mg/kg LY2922470 was the most obvious.

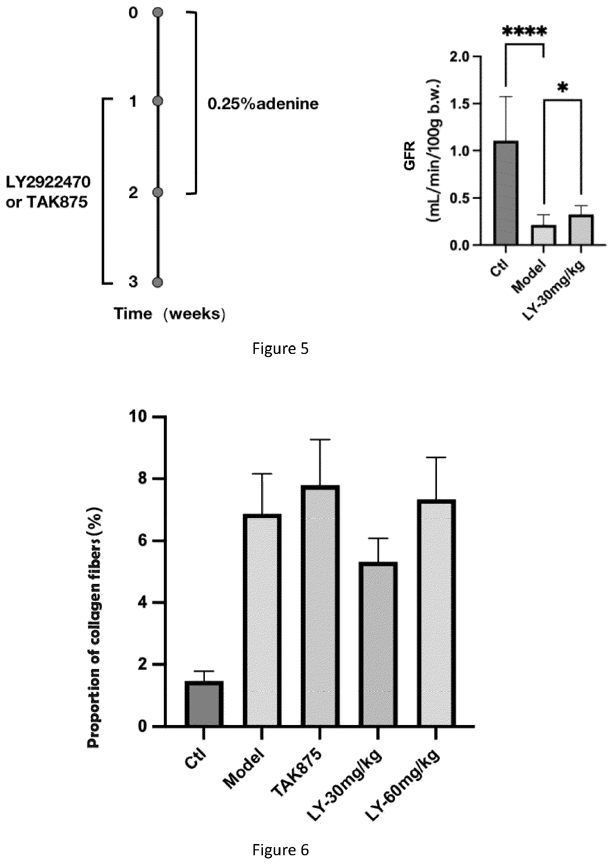

The renal failure of the mice modeled by continuous feeding of 0.25% adenine feed was too severe, resulting in severe renal injury. Therefore, the modeling method was redesigned to verify the therapeutic effect of LY2922470 on renal failure (after 2 weeks of adenine feed, the mice were fed with normal feed, and the rest remained unchanged). The results are shown in figure 5. Compared with the mice in the model group, the renal function of the mice in the LY2922470 drug group was significantly improved, and it was statistically significant (NS: no statistical difference, *p<0.5, **p<0.01, ***p<0.001). The results showed that LY2922470 has a therapeutic effect on renal injury and renal failure.

### ii. Renal Function Indices, Serum Creatinine (CREA) and Urea Nitrogen (BUN)

Mice were fed with 0.25% adenine. One week later, they were treated with LY2922470 or TAK875. During the administration period, they continued to be fed with adenine feed for modeling. After three weeks of adenine feeding (two weeks of administration), the blood of the mice was collected, then the serum was isolated to detect CREA and BUN. The results of CREA and BUN renal function indices in each group of mice are shown in Tables 2 and 3.

There was no significant difference in renal function among mice fed with adenine for one week. After three weeks of modeling (with two weeks of drug administration), compared with mice in the blank control group, CREA and BUN in the model group were upregulated (compared with the blank control group #p<0.05, ##p<0.01). Compared with mice in the model group, CREA and BUN did not decrease significantly after oral administration of the GPR40 agonist TAK875 (compared with the model control group *p<0.5, **p<0.01, ***p<0.001). After oral administration of different doses of LY2922470, the renal function indices of mice, serum creatinine and urea nitrogen, were downregulated. Compared with mice in the model group, the serum CREA of mice in the 30mg/kg and 60mg/kg LY2922470 drug groups was significantly decreased, and the serum BUN of mice in the 30 mg/kg LY2922470 drug group was downregulated with statistical significance. The results showed that LY2922470 has a therapeutic effect on renal injury and renal failure, and this therapeutic effect is not directly related to the GPR40 target.

It can be known from the data in the table that LY2922470 has therapeutic effects at doses ranging from 30 mg/kg/d to 60 mg/kg/d, and has the best therapeutic effect at 30 mg/kg/d. However, it cannot be ruled out that at doses <30 mg/kg/d (for example: The protective effect of 20-30 mg/kg on the kidneys cannot be ruled out, and the therapeutic effect of doses >60 mg/kg/d, such as 60-100 mg/kg, on renal diseases cannot be excluded.

**Table 2: Renal Function Index CREA of mice in each group**

| Group | CREA (µmol/L) | |
|---|---|---|
| | Modeling 1 week (before treatment) | Modeling 3 weeks (after 2 weeks of treatment) |
| Blank Control Group | 53.74 ± 14.79 | 59.25 ± 29.55 |
| Model Group | 53.87 ± 14.56 | 159.75 ± 14.42 |
| TAK Group | 46.54 ± 11.22 | 137.68 ± 30.23 |
| LY-10 Group | 56.14 ± 13.41 | -- |
| LY-30 Group | 54.85 ± 17.55 | 79.67 ± 19.28** |
| LY-60 Group | 57.02 ± 11.90 | 66.47 ± 13.35** |

**Table 3: Renal Function Index BUN of mice in each group**

| Group | BUN (mmol/L) | |
|---|---|---|
| | Modeling 1 week (before treatment) | Modeling 3 weeks (after 2 weeks of treatment) |
| Blank Control Group | 11.36 ± 3.64 | 12.70 ± 8.17 |
| Model Group | 10.63 ± 3.47 | 24.72 ± 11.83 |
| TAK Group | 11.25 ± 3.98 | 25.07 ± 4.91 |
| LY-10 Group | 10.34 ± 3.55 | - |
| LY-30 Group | 10.25 ± 3.42 | 15.15 ±4.53* |
| LY-60 Group | 10.40 ± 3.83 | 13.85 ± 1.33 |

### Embodiment 4: Inhibitory Effect of LY2922470 on Renal Fibrosis

### I. Experimental materials

### Same as embodiment 3

### II. Experimental groups

### Same as embodiment 3

### III. Experimental methods

### i. Experimental Procedures

Modeling and drug administration were performed according to the method of embodiment 3. After 2 weeks of drug administration, mice were euthanized, and kidneys were fixed, sliced, and stained with MASSON for analysis.

### ii. Data Collection and Statistical Analysis

The slides were scanned using the fully automatic digital pathology slide scanner KF-PRO-120 from Ningbo Jiangfeng Bioinformatics Co., Ltd. The Masson staining images were analyzed using the Area Quantification module of the HALO software. The blue color of collagen fiber staining was selected as the uniform standard for positive, the images were analyzed, and the percentage of blue collagen fibers in the tissue was calculated.

GraphPad software and Prism 8.0 statistical software were used for statistical analysis. The data of each group of measurement data were expressed as mean ± SD. ANOVA one-way analysis of variance was used for multiple group comparisons. The t-test was used for comparisons between two groups. P < 0.05 was considered to be statistically significant. The chi-square test was used for comparisons between groups of count data. P < 0.05 was considered to be statistically significant.

### IV. Experimental Results and Conclusions

As shown in figure 6, after three weeks of modeling (two weeks of administration), the degree of renal fibrosis in the model group mice was significantly increased compared with the blank control group mice. Compared with the model group mice, the degree of fibrosis did not decrease significantly after oral administration of the GPR40 agonist TAK875. After the mice were orally administered LY2922470 at a dose of 30 mg/kg/d, the amount of collagen deposition decreased, ECM deposition was inhibited, and the degree of renal fibrosis was alleviated. The experimental results show that LY2922470 can resist renal fibrosis, and the anti-fibrosis can act independently of the GPR40 target.

### Embodiment 5: Inhibitory Effect of LY2922470 on Renal Fibrosis (TGF-β1 induced fibrosis)

### I. Experimental Materials

| Name | Manufacturer | Catalog Number |
|---|---|---|
| HK-2 Cells | Pnoxai | CL-0109 |
| MEM Culture Medium | Pnoxai | PM150410 |
| FBS | Lonsera | |
| Penicillin-Streptomycin Solution | Biosharp | BL505A |
| 0.25% Trypsin | CTCC | 002PI |
| PBS Phosphate Buffered Saline | CTCC | 013PI |
| Other Chemical Reagents | CN Domestic Analytical Pure | |

### II. Experimental Grouping

A: Blank Control Group.
B: TGF-β1 (10 ng/ml).
C: TGF-β1 (10 ng/ml) + LY (10 nM).
D: TGF-β1 (10 ng/ml) + LY (50 nM).
E: TGF-β1 (10 ng/ml) + LY (500 nM).

### III. Experimental Methods

HK-2 cell culture medium: MEM +10%FBS+1% (Penicillin-Streptomycin Solution). Take out HK-2 cells from liquid nitrogen, quickly put them into a 37°C water bath, and gently shake the cryopreservation tube to dissolve the cryopreservation solution; after dissolution, transfer the cells to a centrifuge tube containing 3 mL of culture medium, collect the cells by centrifugation, centrifuge at 1000 rpm for 5 min at room temperature, and discard the supernatant; suspend the cells with complete culture medium containing 10% fetal bovine serum, inoculate them into a culture dish, gently blow and mix, and culture at 37 °C and 5% CO2 saturated humidity.

HK-2 cells in the logarithmic growth phase and in good growth condition were taken and inoculated into a 24-well plate at 1×105 cells/well; after the cells reached a density of 40%-50%, the cells were treated according to the above groups: Group A HK-2 was cultured normally; Group B was treated with 10 ng/ml TGF-β1; Group C was treated with 10 ng/ml TGF-β1 and 10 nM LY; Group D was treated with 10 ng/ml TGF-β1 and 50 nM LY; Group E was treated with 10 ng/ml TGF-β1 and 500 nM LY; each group was cultured for 48 hours and then samples were collected for RT-PCR detection. The primer sequences are shown in the table below.

| **Primer** | **Sequence (5'-3')** |
|---|---|
| Fibronectin (human) -RT-F | GAAGTGGGACCGTCAGGGAGAAA |
| Fibronectin (human) -RT-R | CAGGAGCAAATGGCACCGAGATA |
| α-SMA (human) -RT-F | CCCCTAAACCCTAAAGCCAACA |
| α-SMA (human) -RT-R | GCAGTGCATAGCCCTCGTAGAT |
| Gapdh (human) -RT-F | GGAGCGAGATCCCTCCAAAAT |
| Gapdh (human) -RT-R | GGCTGTTGTCATACTTCTCATGG |

### IV. Experimental Results

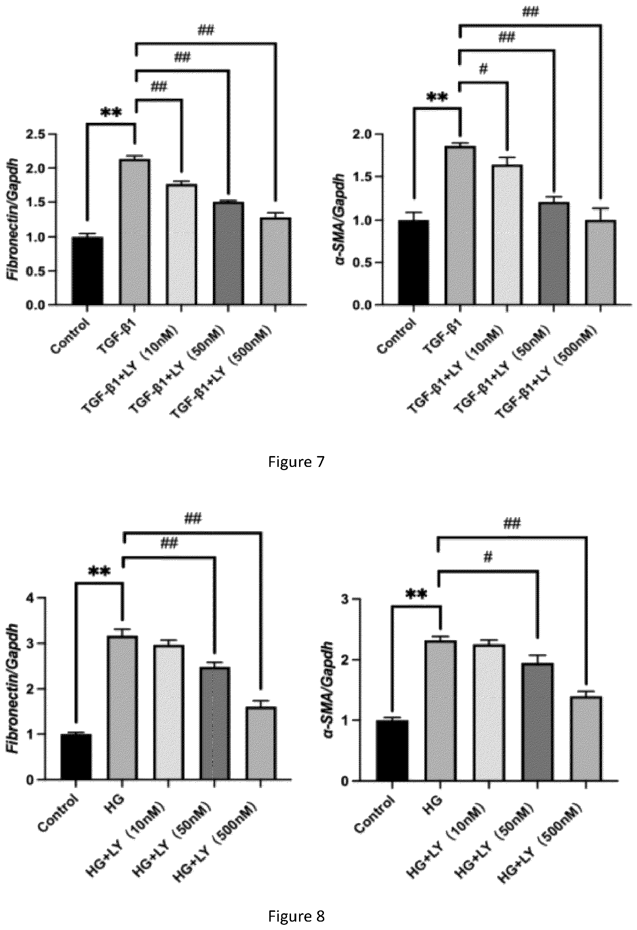

The anti-fibrotic effect of LY2922470 was confirmed using the TGF-β1-induced human renal cortical proximal tubule epithelial cell HK-2 cell model. The results of the RT-PCR experiment showed (Figure 7): Compared with the blank control group, the addition of TGF-β 1 treatment significantly increased the expression levels of fibrosis markers Fibronectin and α-SMA; compared with the TGF-β1 group, the addition of different concentrations of LY treatment reduced the expression levels of Fibronectin and α-SMA to varying degrees, and was dose-dependent with the added concentration (*: compared with the blank control group, 0.01<p<0.05; **: compared with the blank control group, p<0.01; #: compared with the TGF-β1 group, 0.01<p<0.05; ##: compared with the TGF-β1 group, p<0.01).

### Embodiment 6: Inhibitory Effect of LY2922470 on Renal Fibrosis (High Glucose Induced Fibrosis)

### I. Experimental Materials

i. D-glucose (purchased from Shanghai Guoyao).
ii. The rest is the same as embodiment 5.

### II. Experimental Grouping

A: Blank Control Group.
B: HG (25 mM).
C: HG (25 mM) + LY (10 nM).
D: HG (25 mM) + LY (50 nM).
E: HG (25 mM) + LY (500 nM).

### III. Experimental Methods

HK-2 cell culture medium: MEM +10%FBS+1% (Penicillin-Streptomycin Solution). Take out HK-2 cells from liquid nitrogen, quickly put them into a 37 °C water bath, and gently shake the cryopreservation tube to dissolve the cryopreservation solution; after dissolution, transfer the cells to a centrifuge tube containing 3 mL of culture medium, collect the cells by centrifugation, centrifuge at 1000 rpm for 5 min at room temperature, and discard the supernatant; suspend the cells with complete culture medium containing 10% fetal bovine serum, inoculate them into a culture dish, gently blow and mix, and culture at 37 °C and 5% CO₂ saturated humidity.

After the cells reached a density of 40%-50%, the cells were treated according to the above groups: Group A HK-2 was cultured normally; Group B was treated with 25 mM D-glucose high glucose; Group C was treated with 25mM D-glucose high glucose and 100nM TAK; Group D was treated with 25 mM D-glucose high glucose and 10nM LY; Group E was treated with 25 mM D-glucose high glucose and 50nM LY; Group F was treated with 25 mM D-glucose high glucose and 500 nM LY; each group was cultured for 48 h and then samples were collected for RT-PCR detection.

### IV. Experimental Results

The HK-2 injury model was induced using high glucose medium to confirm the anti-fibrotic effect of LY2922470. The results of RT-PCR experiments showed (Figure 8): Compared with the blank control group, high glucose treatment significantly increased the expression levels of Fibronectin and α-SMA; compared with the high glucose group, the addition of different concentrations of LY treatment reduced the expression levels of Fibronectin and α-SMA to varying degrees, and was dose-dependent with the added concentration (*: compared with the blank control group, 0.01<p<0.05; **: compared with the blank control group, p<0.01; #: compared with the high glucose group, 0.01<p<0.05; ##: compared with the high glucose group, p<0.01).

The present invention evaluates the effect of LY2922470 and TAK875 on the treatment of renal diseases by using adenine-induced renal injury, renal failure animal models and renal fibrosis in vitro models. The experimental results show that LY2922470 has the effect of protecting renal function, treating renal injury, chronic renal diseases, renal failure and other diseases, and the therapeutic effect has no direct relationship with the GPR40 target. The in vitro models of TGF-β1-induced fibrosis and high sugar-induced fibrosis confirm that LY2922470 has an anti-fibrotic effect. Therefore, the present invention confirms the great potential of LY2922470 as a drug for renal diseases, and LY2922470 has the effect of treating renal diseases and has broad application prospects in clinical practice.

## Claims

1. A use of LY2922470 in preparing a medicament for preventing or treating renal diseases, wherein the LY2922470 comprising following structural formula:

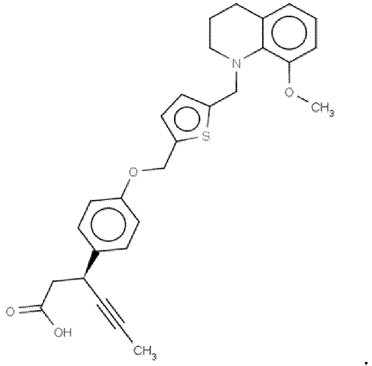

2. The use according to claim 1, wherein the renal diseases comprise renal injury, chronic rental diseases, or renal failure

3. The use according to claim 1, wherein the renal diseases comprise renal diseases caused by renal fibrosis.

4. The use according to claim 2, the renal diseases comprise nephropathy caused by ischemia-reperfusion (IR), nephrotoxins, chemotherapeutic medicaments, hypertension or ureteral obstruction or diabetic nephropathy.

5. The use according to claim 1, wherein the medicament comprises the LY2922470 exists in a form of a compound or a pharmaceutically acceptable salt thereof.

6. The use according to claim 1, wherein the medicament is in an oral or injectable dosage form, which comprises powders, tablets, granules, capsules, oral liquids, emulsions or suspensions.

7. The use according to claim 1, wherein the medicament further comprises auxiliary materials or medicines for protecting renals and blood vessels.

8. The use according to claim 1, wherein the medicament further comprises a pharmaceutically acceptable carrier, and the pharmaceutically acceptable carrier comprises a diluent, a buffer, a suspension, an emulsion, a granule, an encapsulation, an excipient, a filler, an adhesive, a spray, a transdermal absorbent, a wetting agent, a disintegrant, an absorption accelerator, a surfactant, a colorant, a flavoring agent or an adsorption carrier.
